# EUROPEAN PATENT SPECIFICATION

(11) **EP 2 265 247 B1**
(45) Date of publication and mention of the grant of the patent: **03.05.2017**
(21) Application number: 09722126.1
(22) Date of filing: 20.03.2009
(51) Int. Cl.: A61K 8/44, A61K 8/90, A61Q 11/00

(54) **COMPOSITIONS COMPRISING NONIONIC AND ZWITTERIONIC SURFACTANTS**
NICHTIONISCHE UND ZWITTERIONISCHE TENSIDE UMFASSENDE ZUSAMMENSETZUNGEN
COMPOSITIONS COMPRENANT DES TENSIOACTIFS NON IONIQUES ET ZWITTÉRIONIQUES

(30) Priority: 21.03.2008 US 53222
(43) Date of publication of application: 29.12.2010
(73) Proprietor: Colgate-Palmolive Company, New York, NY 10022 (US)
(72) Inventor: JOZIAK, Marilou, South River, NJ 08882 (US); NESTA, Jason, Cedar Knolls, NJ 07927 (US)
(74) Representative: Wibbelmann, Jobst
(86) International application number: PCT/US2009/037777
(87) International publication number: WO 2009/117643

(56) References cited:
- EP-A- 1 437 126
- WO-A-02/26203
- WO-A-95/01154
- WO-A-99/63959
- WO-A-2008/145475
- WO-A-2009/099455
- FR-A- 2 341 302
- JP-A- H1 087 457
- US-A- 4 774 077
- US-A- 5 071 637
- US-A- 5 073 368
- SODERLING E ET AL: "Betaine-containing toothpaste relieves subjective symptoms of dry mouth" ACTA ODONTOLOGICA SCANDINAVICA, OSLO, NO, vol. 56, no. 2, 8 June 1998 (1998-06-08), pages 65-69, XP008107759 ISSN: 0001-6357

## Description

### BACKGROUND OF THE INVENTION

Foaming agents containing anionic surfactants, such as sodium lauryl sulfate, sodium laureth sulfate, and sodium methyl cocoyl taurate have traditionally been used in consumer care products, oral products, e.g., dentifrices, due to their low cost and ability to foam. The foaming ability of a consumer product is important in that consumers correlate the foaming ability with the effectiveness of the product. However, many people suffer from adverse reactions to anionic surfactants. For example, anionic surfactants tend to irritate the oral cavity, and may cause discomfort and irritation, especially in patients suffering from various oral conditions, e.g., xerostomia and/or tooth sensitivity. Such irritation is especially undesirable, especially in people suffering from, or predisposed to various conditions, such as xerostomia (dry mouth). Current products used for the treatment of dry mouth require high concentrations of fluoride to lower their risk of developing caries. However, such products may not recognize that the ingredients contained therein may cause irritation of the oral cavity, and thus exacerbate xerostomia.

Removing anionic surfactants from consumer products reduces their ability to produce foam. As previously stated, the ability of a product to foam is important in that consumers may correlate the foaming ability of a composition with its efficacy. Moreover, the foam produced by an oral care product may containing active ingredients, and the foam allows for the active ingredient to permeate throughout the oral cavity, including areas between teeth which are not accessible by brushing alone. Previous attempts to develop anionic surfactant free consumer products which produce an acceptable quality and quantity of foam has failed, as surfactants are incompatible with the product, or the rheology of the product must be modified in order to incorporate replacement surfactants. In many cases, such modifications render the composition unsuitable for its intended use.

Thus, there is a need to develop anionic surfactant free foaming agents. There is also a need to develop oral products which do not contain irritating ingredients, or reduces irritating ingredients present in the composition. Such compositions may be utilized to treat and/or alleviate xerostomia.

### BRIEF SUMMARY OF THE INVENTION

The present invention is directed to the suprising discovery that a foaming agent comprising nonionic and zwitterionic surfactants may be used to replace foaming agents containing anionic surfactants. A foaming agent comprising a non-ionic surfactant, a zwitterionic surfactant, and which is free of anionic surfactants is therefore described herein. The foaming agent may be used in any number of personal care products, such as oral products, e.g., dentifrice compositions. The ability for such compositions to foam is equivalent to, or may even be superior to similar compositions containing one or more anionic surfactants.

Thus, described herein is Foaming Agent 1.0, comprising a non-ionic surfactant and a zwitterionic surfactant, wherein the foaming agent is free of anionic surfactants, e.g., SDS, SLS, or sodium methylcocoyl taurate;
wherein the non-ionic surfactant is a poly(oxyethylene)-poly(oxypropylene) copolymer;
wherein the foaming agent comprises from from 0.5% to 10% of a non-ionic surfactant, e.g.1%, 2%, or 3%; and
wherein the zwitterionic surfactant is an amido betaine; and wherein the foaming agent comprises a zwitterionic surfactant, e.g., 0.2 - 5%, 0.4%, 0.5%, 0.6%, or 1%.

Additionally described herein are the following compositions:
1.1. Any of the preceding foaming agents wherein the zwitterionic surfactant is cocoamidoethyl betaine, cocoamidopropyl betaine, lauramidopropyl betaine, or combinations thereof;
1.2. Any of the preceding foaming agents wherein the zwitterionic surfactant is cocoamidopropyl betaine.
1.3. Any of the preceding foaming agents which produces an equivalent quantity of foam compared to a comparison agent which comprises an anionic surfactant and is substantially free of non-ionic and zwitterionic surfactants;
1.4. Any of the foaming agents which produces an equivalent quality of foam compared to a comparison agent which comprises an anionic surfactant and is substantially free of non-ionic and zwitterionic surfactants;
1.5. Foaming agents 1.5 and 1.4 wherein the comparison agent comprises about 1.2% sodium methyl cocoyl taurate.

Also described herein is Composition 2.0, a dentifrice composition comprising any one of foaming agents 1.0-1.5.

The present invention provides a dentifrice composition according to claim 1, comprising (i) a foaming agent comprising a nonionic surfactant and a zwitterionic surfactant, wherein the foaming agent is free of anionic surfactants, the nonionic surfactant is a poly(oxyethylene)-poly(oxypropylene) copolymer, and the zwitterionic surfactant is an amido betaine; and (ii) an effective amount of a halogenated diphenyl ether or cetylpyridinium chloride; wherein anionic surfactants have not been added to the composition; wherein the foaming agent comprises from 0.5% to 10% of the non-ionic surfactant; and wherein the foaming agent comprises from 0.1% to 10% of the zwitterionic surfactant. The present invention is also directed to the following compositions:
2.1 Any of the preceding compositions further comprising a fluoride ion source, e.g., sufficient to provide from about 250 ppm to about 25,000 ppm fluoride ions, e.g., about 1000 ppm to about 1500 ppm, about 1250 ppm, 1500 ppm, or about 5000 ppm fluoride ions.
2.2 Composition 2.1 wherein the fluoride ion source is sodium fluoride, potassium fluoride, calcium fluoride, zinc fluoride, zinc ammonium fluoride, lithium fluoride, ammonium fluoride, stannous fluoride, stannous fluorozirconate, sodium monofluorophosphate, potassium monofluorophosphate, laurylamine hydrofluoride, diethylaminoethyloctoylamide hydrofluoride, didecyldimethylammonium fluoride, cetylpyridinium fluoride, dilaurylmorpholinium fluoride, sarcosine stannous fluoride, glycine potassium fluoride, glycine hydrofluoride, amine fluorides, or combinations thereof;
2.3 Any of the preceding compositions comprising an effective amount of a desensitizing agent;
2.4 Any of the preceding compositions comprising a potassium salt, capsaicin, eugenol, a strontium salt, a zinc salt, a chloride salt, or combinations thereof;
2.5 Any of the preceding compositions comprising potassium nitrate, potassium bicarbonate, potassium chloride, potassium citrate, potassium oxalate, or combinations thereof;
2.6 Any of the preceding compositions comprising an effective amount of an antibacterial agent;
2.7 Any of the preceding compositions wherein the halogenated diphenyl ether is triclosan;
2.8 Any of the preceding compositions comprising stannous ion agent, triclosan, triclosan monophosphate, chlorhexidine, alexidine, hexetidine, sanguinarine, benzalkonium chloride, salicylanilide, arginate esters, ethyl lauryl arginate, bisphenols, domiphen bromide, tetradecylpyridinium chloride, N-tetradecyl-4-ethylpyridinium chloride, octenidine, delmopinol, octapinol, nisin, zinc ion agent, copper ion agent, essential oils, furanones, bacteriocins, a basic amino acid, or combinations thereof;
2.9 Any of the preceding compositions comprising silica, a humectant, a stabilizing agent, a sweetener, a surfactant, or combinations thereof;
2.10 Any of the preceding compositions which is a dentifrice, e.g., a toothpaste;
2.11 Any of the preceding compositions comprising 0.5% of cocoamidopropyl betaine;
2.12 Any of the preceding compositions comprising 2% of a poly(oxyethylene)-poly(oxypropylene) copolymer;

The present invention is also directed to a method for treating and/or alleviating xerostomia comprising applying any one of the preceding compositions to the oral cavity, e.g., by brushing.

Other embodiments of the present invention will be apparent to one of skill in the art.

### DETAILED DESCRIPTION OF THE INVENTION

Throughout the present disclosure, ranges are used as a shorthand for describing each and every value that is within the range. Any value within the range can be selected as the terminus of the range.

In an embodiment of the present invention, a foaming agent comprising zwitterionic and non-ionic surfactants, and free of anionic surfactants is provided. Anionic surfactants are not added to the foaming agent, although some may be present due to unintended contamination, or decomposition of other ingredients, or unintended reaction between other ingredients. Anionic surfactants are known by those of skill in the art.

Zwitterionic surfactants are known in the art, and generally include surfactants which are neutrally charged overall, but carry at least one positive charged atom/group, and at least one negatively charged atom/group. Examples of zwitterionic surfactants are quaternary ammonium compounds and betaines. The zwitterionic surfactants used in the present invention are amido betaines, such as cocoamidoethyl betaine, cocoamidopropyl betaine, lauramidopropyl betaine and the like. A preferred zwitterionic surfactant for use in the present invention is the cocoamidopropyl betaine.

Non-ionic surfactants are known in the art, and generally include surfactants which are not electrically charged. In the compositions of the present invention, the nonionic surfactant is poly(oxyethylene)-poly(oxypropylene) block copolymers. Such copolymers are known commercially by the non-proprietary name of poloxamers. The name poloxamer is often used in conjunction with a numeric suffix to designate the individual identification of each copolymer. Poloxamers may have varying contents of ethylene oxide and propylene oxide which results in poloxamers which have a wide range of chemical structures and molecular weights. A preferred poloxamer is Poloxamer 407, sold under the trade name PLURONIC F 127 by BASF, Inc. (Parsippany, N.J.).

The foaming agent may be incorporated into personal care compositions, e.g., compositions for application on the skin or oral cavity. In the compositions of the present invention, the foaming agent is incorporated into an oral care composition, which may also include a fluoride ion source, an antibacterial agent, and/or an anti-sensitivity agent. Anionic surfactants are not added to the composition, although some may be present due to unintended contamination, or decomposition of other ingredients, or unintended reaction between other ingredients.

The oral care composition is in the form of a dentifrice. Preferred dentifrices include but are not limited to various types of toothpaste, tooth polish, tooth gel, mouthwash and mouth rinse, denture adhesive or cream or the like.

The oral care compositions of the present invention preferably contain fluoride ions. Fluoride ions may be provided by a fluoride ion source. A fluoride ion source may be anything that is capable of releasing fluoride ion in an aqueous environment. Typical sources include soluble salts of the fluoride ion; such as, for example: sodium fluoride, potassium fluoride, calcium fluoride, zinc fluoride, zinc ammonium fluoride, lithium fluoride, ammonium fluoride, stannous fluoride, stannous fluorozirconate, and complex fluorides, monofluorophosphates and salts thereof such as, *e.g*., sodium monofluorophosphate or potassium monofluorophosphate, laurylamine hydrofluoride, diethylaminoethyloctoylamide hydrofluoride, didecyldimethylammonium fluoride, cetylpyridinium fluoride, dilaurylmorpholinium fluoride, sarcosine stannous fluoride, glycine potassium fluoride, glycine hydrofluoride, and amine fluorides.

The fluoride ion source is most preferably in an amount such that it is capable of maintaining a high level of fluoride ion in the composition that is at least about 250 ppm, and in some instances up to as much as about 25,000 ppm. Preferably, the fluoride ions are present in an amount of about 1000 ppm to about 1500 ppm. In order to provide such a concentration in the optimal ppm range, the exact weight percentage of the fluoride ion source in the composition may vary, depending upon the stoichiometric properties of different fluoride ion sources.

In one embodiment, an oral care composition is provided that can incorporate high levels of fluoride for anticaries treatment, tooth sensitivity, and xerostomia. The compositions may also incorporate one or more sensitivity treatment agents, e.g., potassium salts such as potassium nitrate, potassium bicarbonate, potassium chloride, potassium citrate, and potassium oxalate; capsaicin; eugenol; strontium salts; zinc salts; chloride salts and combinations thereof. Such agents may be added in effective amounts, which preferably vary between 0.01% to 10% by weight based on the total weight of the composition, depending on the agent chosen.

Preferably, a desensitizing agent is a potassium salt in an amount of at least 5% by weight of a potassium salt based on the total weight of the composition. Most preferably, the composition includes 5% to 10% by weight of the potassium salt. Effective amounts of such potassium salts and their use are described in *e.g.,* the following patents: U.S. Patents Nos. 3,863,006, 4,631,185 and 4,751,072. Most preferably, the desensitizing agent is potassium nitrate.

Other thickening agents that may be used in the oral care compositions of the present invention. Other thickening agents are known in the art, and include, but are not limited to: silica thickeners; glycerites; gums such as tragacanth, ghatti, acacia, veegum; sodium alginate; carboxymethyl cellulose; hydroxyethyl cellulose, hydroxypropyl cellulose; hydroxymethyl cellulose; hydroxymethyl carboxypropyl cellulose; methyl cellulose; ethyl cellulose; sulfated cellulose; as well as mixtures and combinations of these compounds. Such additional thickeners may be used in amounts of up to 15 wt % of the composition based on the total weight of the composition.

The oral care compositions of the present invention may also include a variety of common additional active agents typically used in oral care formulations, including but not limited to: triclosan; triclosan monophosphate; chlorhexidine; alexidine; hexetidine; sanguinarine; benzalkonium chloride; salicylanilide; arginate esters; ethyl lauryl arginate, bisphenols, domiphen bromide; tetradecylpyridinium chloride; N-tetradecyl-4-ethylpyridinium chloride; octenidine; delmopinol; octapinol; nisin; zinc ion agent; copper ion agent; essential oils; furanones; bacteriocins; salts of the foregoing active agents and mixtures and combinations thereof.

Optional additives for the oral care compositions of the present invention may also be used, such as those commonly used for forming a dentifrice, including but not limited to: abrasives and/or amorphous silica, humectants, stabilizing agents, antibacterial agents, sweeteners, colorants, healing agents, other caries preventative agents, chelating/sequestering agents, vitamins, amino acids, proteins, anti-plaque agents, anti-calculus agents, opacifiers, antibiotics, anti-enzymes, enzymes, pH control agents, oxidizing agents, antioxidants, whitening agents, basic amino acids (in free base or salt form) and preservatives.

One or more abrasive or polishing materials may also be included in the oral care compositions of the present invention. The abrasive or polishing material can be any material that is acceptable for use in a dentifrice, does not excessively abrade dentin and is compatible with the other components of the oral care composition. Exemplary abrasive or polishing materials include, but are not limited to: silicas, aluminas, phosphates, carbonates, and mixtures, derivatives and salts thereof, and resinous abrasive materials.

One or more humectants may be added to the oral care compositions of the present invention, for providing body or texture to the formulations and for maintaining moisture in the formulations. Useful humectants include, but are not limited to: various polymeric glycols and other hydroxy-based humectants such as, *e.g.*, propylene glycols, glycerol, erythritol, xylitol, sorbitol, mannitol, lactitol, hydrogenated starch hydrolyzates and combinations of these components.

Antibacterial agents can be used if reduction of microorganisms is desired, and can include known antibacterial agents used in dentifrice formulations such as, *e.g*., benzoic acid, sodium benzoate, potassium benzoate, boric acid, and phenolic compounds such as betanaphthol, chlorothymol, thymol, anethole, eucalyptol, carvacrol, menthol, phenol, amylphenol, hexylphenol, heptylphenol, octylphenol, hexylresorcinol, laurylpyridinium chloride, myristylpyridinium chloride, cetylpyridinium fluoride, cetylpyridinium chloride and cetylpyridinium bromide. The compositions of the present invention comprise cetylpyridinium chloride or a halogenated diphenyl ether.

Sweeteners may be used in the oral care compositions of the present invention if desired, and may include any of those commonly used in a dentifrice to impart a pleasing taste to the product. Suitable sweeteners include but are not limited to: saccharins and derivatives thereof, cyclamates and derivatives thereof, acesulfane-K, thaumatin, neohisperidin dihydrochalcone, ammoniated glycyrrhizin, dextrose, levulose, sucrose, mannose, glucose and any other suitable sweeteners.

One of skill in the art would recognize that an ingredient may serve various purposes in the composition. For example, cetylpyridinium chloride may serve as a cationic surfactant, and as an antibacterial agent. Compositions of the present invention may incorporate cetylpyridinium chloride, preferably in amounts effective as an antibacterial agent, though may also act as a surfactant.

The other ingredients noted may be any of those commonly used in dentifrice formulations and can be selected based upon the intended end use of the formulations and to optimize the physical and aesthetic characteristics of the formulations.

The foaming agent of the present invention may also be incorporated into any number of personal care compositions, e.g., lotions, creams, shampoos, conditioners, hair gel, soaps, body wash, or any other personal care product which traditionally utilizes one or more anionic surfactants. The foaming agent of the present invention may also be incorporated into any number of products used to clean a home, or contents therein, e.g., laundry detergent, dish washing detergent, or any other product which utilizes one of more anionic surfactants. Such compositions would be beneficial for use by consumers who suffer from irritations caused by anionic surfactants, such as allergic reactions, or chemical sensitivity.

The invention will now be described with respect to the following non-limiting examples:

### EXAMPLE 1

Four dentifrice compositions are prepared with the formulations provided in Table 1 (numbers are expressed in percent weight). Compositions A1, A2 and Z1, Z2 differ in type of surfactant present (anionic vs. nonionic and zwitterionic respectively). Compositions A1, Z1 and A2, Z2 differ in the amounts and type of flavor present.

**Table 1**

| Ingredient | **A1** | **Z1** | **A2** | **Z2** |
|---|---|---|---|---|
| Glycerin | 3 | 3 | 3 | 3 |
| Xanthan gum | 0.8 | 0.8 | 0.8 | 0.8 |
| Propylene glycol | 5 | 5 | 5 | 5 |
| Sorbitol | 23.8 | 19.7 | 23.8 | 19.7 |
| Sodium fluoride | 1.105 | 1.105 | 1.105 | 1.105 |
| Sodium saccharin | 0.35 | 0.35 | 0.35 | 0.35 |
| Sodium benzoate | 0.5 | 0.5 | 0.5 | 0.5 |
| Potassium sorbate | 0.1 | 0.1 | 0.1 | 0.1 |
| Cetylpyridinium chloride | | 0.1 | | 0.1 |
| Dibasic potassium phosphate | 2 | 2 | 2 | 2 |
| Sodium hydroxide | | | 0..3 | 0.3 |
| Polyoxyl 40 hydrogenated castor oil | 3 | 3 | 3 | 3 |
| Zeodent 165 | 3 | 3 | 3 | 3 |
| Zeodent 114 | 8 | 8 | 8 | 8 |
| Cocoamidopropyl betaine | | 0.45 | | 0.45 |
| Poloxamer 407 | | 2 | | 2 |
| Sodium methyl cocoyl taurate | 1.2 | | 1.2 | |
| Flavor | 1 | 1 | 4.4 | 5.4 |
| FD&C Blue #1 | trace | trace | trace | trace |
| Water | QS 100 | QS 100 | QS 100 | QS 100 |

### EXAMPLE 2

The compositions prepared in Table 1 are provided to an evaluation group, and the group is asked to evaluate the compositions based on foaming characteristics on a scale of 1(low) to 5 (high). The groups are asked to rank the compositions based on quantity and quality of the foam produced. The percentage of users assigning the top two scores (4 and 5) is determined as a "top 2 box" rating, and assigning the bottom two scores (land 2) is determined as a "bottom 2 box". The top2 boxes are presented in Tables 2 and 3.

**Table 2 - Quantity - Foam evaluation**

| Composition | |
|---|---|
| A1 | 58.8%^{a} |
| Z1 | 46.5%^{a} |
| A2 | 61.3%^{b} |
| Z2 | 52.3%^{b} |

| | |
|---|---|
| a - Comparison of quantity of foam produced between A1 and Z1 are not statistically significant b - Comparison of quantity of foam produced between A2 and Z2 are not statistically different | |

**Table 3 - Quality - Foam evaluation**

| Composition | |
|---|---|
| A1 | 58.8%^{c} |
| Z1 | 46.5%^{c} |
| A2 | 57.7%^{d} |
| Z2 | 44.1%^{d} |

| | |
|---|---|
| c - Comparison of quality of foam between A1 and Z1 are not statistically different d - Comparison of quality of foam between A2 and Z2 are not statistically different. | |

Surprisingly, the results indicate that the quantity of foam produced in compositions containing nonionic and zwitterionic surfactants (and free of anionic surfactants) is equivalent to the quantity of foam produced in compositions containing an anionic surfactant. Surprisingly, the results also indicate that the quality of foam produced in compositions containing nonionic and zwitterionic surfactants is equivalent to the quantity of foam produced in compositions containing an anionic surfactant.

It will be appreciated by those skilled in the art that changes and alterations may be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A dentifrice composition comprising: (i) a foaming agent comprising a nonionic surfactant and a zwitterionic surfactant, wherein the foaming agent is free of anionic surfactants, the nonionic surfactant is a poly(oxyethylene)-poly(oxypropylene) copolymer, and the zwitterionic surfactant is an amido betaine; and (ii) an effective amount of a halogenated diphenyl ether or cetylpyridinium chloride; wherein anionic surfactants have not been added to the composition; wherein the foaming agent comprises 0.5% to 10% of the non-ionic surfactant; and
wherein the foaming agent comprises 0.1% to 10% of the zwitterionic surfactant.

2. The composition of any preceding claim wherein the zwitterionic surfactant is selected from the group consisting of cocoamidoethyl betaine, cocoamidopropyl betaine, lauramidopropyl betaine, or combinations thereof.

3. The composition of any preceding claim further comprising a fluoride ion source in an amount sufficient to provide from 250 ppm to 25,000 ppm fluoride ions.

4. The composition of any preceding claim further comprising an effective amount of a desensitizing agent, optionally wherein the desensitizing agent is selected from the group consisting of potassium salts, capsaicin, eugenol, strontium salts, zinc salts, chloride salts, or combinations thereof, further optionally wherein the desensitizing agent is selected from the group consisting of potassium nitrate, potassium bicarbonate, potassium chloride, potassium citrate, potassium oxalate, or combinations thereof.

5. The composition of any preceding claim further comprising silica, a humectant, a stabilizing agent, a sweetener, a surfactant, or combinations thereof.

6. The composition of any preceding claim which is a toothpaste.

7. The dentifrice composition of any preceding claim, comprising:
about 0.5% of cocoamidopropyl betaine, and
about 2% of the poly(oxyethylene)-poly(oxypropylene) copolymer,
said dentifrice composition being free of anionic surfactants.

8. The composition of claim 7 further comprising a fluoride ion source and/or a desensitizing agent.

9. A dentifrice composition for use in a method for treating and/or alleviating xerostomia, the method comprising applying the composition to the oral cavity, wherein the dentifrice composition comprises a dentifrice composition according to any one of claims 1 to 8.

10. The dentifrice composition of claim 9 wherein the composition is for use in such a method in which the dentifrice composition is applied to the oral cavity by brushing of the teeth.

## Patentansprüche

1. Eine Zahnpastenzusammensetzung bestehend aus: (i) einem Schaummittel bestehend aus einem nichtionischen und einem zwitterionischen Tensid, wobei das Schaummittel frei von anionischen Tensiden ist, das nichtionische Tensid ein Poly(oxyethylen)-Poly(oxypropylen)-Copolymer ist und das zwitterionische Tensid ein Amidobetain ist; und (ii) einer wirksamen Menge eines halogenierten Diphenylethers oder Cetylpyridiniumchlorids; wobei der Zusammensetzung keine anionischen Tenside hinzugefügt wurden; wobei das Schaummittel 0,5 % bis 10 % des nichtionischen Tensids ausmacht und wobei das Schaummittel 0,1 % bis 10 % des zwitterionischen Tensids enthält.

2. Die Zusammensetzung entsprechend einem der vorherigen Ansprüche, wobei das zwitterionische Tensid aus der Gruppe bestehend aus Cocoamidoethyl-Betain, Cocoamidopropyl-Betain, Lauramidopropyl-Betain oder Kombinationen dieser ausgewählt wird.

3. Die Zusammensetzung entsprechend einem der vorherigen Ansprüche, die zudem eine Fluoridionenquelle in ausreichender Menge umfasst, um 250 ppm bis 25.000 ppm Fluoridionen bereitzustellen.

4. Die Zusammensetzung entsprechend einem der vorherigen Ansprüche, die zudem eine wirksame Menge eines Desensibilierungsmittels umfasst, wobei das Desensibilierungsmittel optional aus der Gruppe bestehend aus Kaliumsalzen, Capsaicin, Eugenol, Strontiumsalzen, Zinksalzen, Chloridsalzen
oder Kombinationen dieser ausgewählt wird, wobei das Desensibilierungsmittel zudem optional aus der Gruppe bestehend aus Kaliumnitrat, Kaliumbicarbonat, Kaliumchlorid, Kaliumcitrat, Kaliumoxalat oder Kombinationen dieser ausgewählt wird.

5. Die Zusammensetzung entsprechend einem der vorherigen Ansprüche, die zudem Siliziumdioxid, ein Feuchthaltemittel, einen Süßstoff oder eine Kombination dieser enthält.

6. Die Zusammensetzung entsprechend einem der vorherigen Ansprüche, bei der es sich um eine Zahnpaste handelt.

7. Die Zahnpastenzusammensetzung entsprechend einem der vorherigen Ansprüche bestehend aus:
ca. 0,5% Cocoamidopropyl-Betain und
ca. 2 % des Poly(oxyethylen)-Poly(oxypropylen)-Copolymers,
wobei die genannte Zahnpastenzusammensetzung frei von anionischen Tensiden ist.

8. Die Zusammensetzung entsprechend Anspruch 7, die zudem eine Fluoridionenquelle und/oder ein Desensibilierungsmittel umfasst.

9. Eine Zahnpastenzusammensetzung zur Verwendung bei einem Verfahren zur Behandlung von Xerostomie, wobei das Verfahren darin besteht, dass die Zusammensetzung in der Mundhöhle angewandt wird, wobei die Zahnpastenzusammensetzung eine Zahnpastenzusammensetzung entsprechend einem der Ansprüche 1 bis 8 ist.

10. Die Zahnpastenzusammensetzung entsprechend Anspruch 9, wobei die Zusammensetzung zur Verwendung in einem Verfahren dient, bei dem die Zahnpastenzusammensetzung in der Mundhöhle durch Zähnebürsten angewandt wird.

## Revendications

1. Composition dentifrice comportant : (i) un agent moussant comportant un tensioactif non ionique et un tensioactif zwittérionique, dans laquelle l'agent moussant est exempt de tensioactifs anioniques, le tensioactif non ionique est un copolymère de poly(oxyéthylène)-poly(oxypropylène), et le tensioactif zwittérionique est une amidobétaïne ; et (ii) une quantité efficace d'un éther de diphényle halogéné ou de chlorure de cétylpyridinium ; dans laquelle des tensioactifs anioniques n'ont pas été ajoutés à la composition ; dans laquelle l'agent moussant comporte de 0,5 à 10 % de tensioactifs non ioniques ; et dans laquelle l'agent moussant comporte de 0,1 à 10 % de tensioactifs zwittérioniques.

2. Composition selon l'une quelconque des revendications précédentes dans laquelle le tensioactif zwittérionique est sélectionné dans le groupe constitué par la cocoamidoéthyl-bétaïne, la cocoamidopropyl-bétaïne, la lauramidopropyl-bétaïne, ou leurs combinaisons.

3. Composition selon l'une quelconque des revendications précédentes comportant en outre une source d'ions fluorure en quantité suffisante pour fournir de 250 à 25 000 ppm d'ions fluorure.

4. Composition selon l'une quelconque des revendications précédentes comportant une quantité efficace d'un agent désensibilisant, facultativement dans laquelle l'agent désensibilisant est sélectionné dans le groupe constitué par les sels de potassium, la capsaïcine, l'eugénol, les sels de strontium, les sels de zinc, les sels de chlorure, ou leurs combinaisons, facultativement en outre dans laquelle l'agent désensibilisant est sélectionné dans le groupe constitué par le nitrate de potassium, le bicarbonate de potassium, le chlorure de potassium, le citrate de potassium, l'oxalate de potassium, ou leurs combinaisons.

5. Composition selon l'une quelconque des revendications précédentes comportant de la silice, un humectant, un agent stabilisant, un édulcorant, un tensioactif, ou leurs combinaisons.

6. Composition selon l'une quelconque des revendications précédentes qui est une pâte dentifrice.

7. Composition dentifrice selon l'une quelconque des revendications précédentes, comportant :
environ 0,5 % de cocoamidopropyl-bétaïne, et
environ 2 % du copolymère de poly(oxyéthylène)-poly(oxypropylène),
ladite composition dentifrice étant exempte de tensioactifs anioniques.

8. Composition selon la revendication 7 comportant en outre une source d'ions fluorure et/ou un agent désensibilisant.

9. Composition dentifrice destinée à être utilisée dans le cadre d'une méthode de traitement et/ou de soulagement de la xérostomie, la méthode consistant à appliquer la composition dans la cavité buccale, la composition dentifrice comportant une composition dentifrice selon l'une quelconque des revendications 1 à 8.

10. Composition dentifrice selon la revendication 9 dans laquelle la composition est destinée à être utilisée dans le cadre d'une méthode par laquelle la composition dentifrice est appliquée dans la cavité buccale par brossage des dents.
